(19) Europäisches Patentamt
European Patent Office
Office européen des brevets

(11) Publication number: 0 119 833 B1

# (12) EUROPEAN PATENT SPECIFICATION

(45) Date of publication of patent specification: 17.11.88

(51) Int. Cl.$^4$: C 07 C 67/08, C 07 C 69/14

(21) Application number: 84301747.6

(22) Date of filing: 14.03.84

## (54) Preparation of carboxylic acid esters of alkylene glycol monoalkyl ethers.

(30) Priority: 17.03.83 US 476305

(43) Date of publication of application:
26.09.84 Bulletin 84/39

(45) Publication of the grant of the patent:
17.11.88 Bulletin 88/46

(84) Designated Contracting States:
BE DE FR GB IT NL

(56) References cited:
DE-A-1 919 527

(73) Proprietor: ATLANTIC RICHFIELD COMPANY
515 South Flower Street
Los Angeles California 90071 (US)

(72) Inventor: Gupta, Vijai P.
816 Newtown Road
Berwyn Pennsylvania 19312 (US)

(74) Representative: Cropp, John Anthony David et al
MATHYS & SQUIRE 10 Fleet Street
London, EC4Y 1AY (GB)

EP 0 119 833 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Background of the invention

The present invention is directed to a novel improved method for the preparation of monocarboxylic acid esters of alkylene glycol monoalkyl ethers such as for example, propylene glycol monomethyl ether acetate by the acid catalyzed esterification of acetic acid with propylene glycol monomethyl ether.

The esters produced by the process of this invention find many important commercial applications and particularly as solvents for inks and coating polymers and in cleansers.

Prior art processes are known for the preparation of glycol ether esters by the acid catalyzed esterification of mono-, di- and triglycol alkyl ethers with a carboxylic acid such as acetic acid. The reaction liberates water which in addition to unreacted reactants (acid and glycol ether) causes operational as well as purification and separation problems. Commonly, the transesterification of ethyl acetate with the glycol ether is employed to produce the glycol ether acetate.

French Demande 2,187,757 describes the continuous preparation of esters of glycol monoethers with lower fatty acids.

U.S. Patent No. 3,404,175 describes a process of forming and separating ethers of esters by plural stage distillation with removal of water as an azeotrope.

German Offenlegungsschrift 1,919,527 shows the continuous production of esters ($AcOCH_2CH_2OMe$ and $AcO(CH_2)_2OEt$) by maintaining the lower portion of the distillation column at > atmospheric pressure.

U.S. 3,637,794 discloses borate esters prepared by successive reactions of boric acid with glycol monoethers and polyols.

Summary of the invention

This invention relates to a method for the preparation of monocarboxylic acid esters of alkylene glycol monoalkyl ethers, and especially the preparation of propylene glycol monomethyl ether acetate, and involves reacting a monocarboxylic acid and a glycol ether in the presence of an acid catalyst with a water azeotroping agent such as toluene. Water added with the reactants and water produced by the esterification reaction is continuously stripped out of the reaction via a distillation column using an inert water-azeotroping solvent. At the same time, the azeotroping solvent is refluxed to the distillation column along with a reflux of the water phase by means of a phase separator, allowing for discharge of some of the water. Enough of the water phase is refluxed along with the azeotropic solvent to lower and maintain the column overhead temperature of the distillation column and thus greatly reduce the loss of reactant glycol ether and carboxylic acid as well as product ester thus obtaining high conversions and high selectivity.

It is therefore an object of this invention to provide an improved method for the preparation of monocarboxylic acid esters of glycol ethers in high yield and selectivity.

It is another object of this invention to lower the overhead temperature of a distillation unit employed in an acid catalyzed esterification reaction of glycol ethers with monocarboxylic acids by azeotroping off water with an azeotroping solvent with reflux of the solvent and at least partial reflux of the water to the distillation unit to provide minimum losses of reactants and product.

These and other objects and advantages of this invention will become apparent from the description and from the claims.

Brief description of the drawings

The figure is a schematic diagram of a specific flow system which can be used to carry out the process of this invention.

Detailed description of the invention and drawing

In accordance with this invention an alkylene glycol monoalkyl ether ester is produced by reacting a monocarboxylic acid with an alkylene glycol monoalkyl ether of the general formulae

$$R(OCH_2CHR')_nOH \quad \text{and} \quad R(O\overset{R'}{\overset{|}{C}}H{-}CH_2)_nOH$$

wherein R is a straight or branched chain alkyl group containing from 1 to 10 carbon atoms, R' is hydrogen or a methyl group and n is an integer of from 1 to 3 at elevated temperatures and preferably atmospheric pressure in the presence of an acid catalyst and a water-azeotroping solvent which forms a binary minimum boiling azeotrop with water within the reaction temperature range, which essentially total reflux of the azeotroping solvent phase and a partial reflux of the water phase to the distillation column. By removing and refluxing at least part of the water as it is formed by the reaction, the overhead temperature of the distillation column is reduced and maintained within a desirable range of from about 65°C. to 95°C. in order to minimize the loss of glycol ether and carboxylic acid reactants as well as ester product in the water distillate. Without at least a partial reflux of the water phase to the distillation column, overhead temperatures generally will increase up to as high as 125°C. depending on the ester being produced.

Lowering or minimizing the amount of azeotroping solvent charged to the reaction system allows the reaction pot temperatures to increase to within a range of from about 80°C. to 165°C., preferably 115°C. to 145°C., thus increasing the reaction rate to permit a reduction in the amount of acid catalyst employed. Generally the amount of azeotroping solvent used will be between about 1 to 25 weight per cent and preferably between 3 to 10 weight per cent of the total reaction mixture.

The acid catalyzed esterification reaction may be carried out in any suitable reactor which may

be equipped with means for agitation, a means for regulating temperature and a means, such as distillation column, a condenser and a phase separator or decanter, for removing the solvent-water azeotrope with a means for returning the azeotroping solvent and the water to the distillation column. A general procedure for carrying out the reaction is to charge the glycol ether, caboxylic acid, azeotroping solvent and acid catalyst into the reaction vessel, and then heat or maintain the mixture at the desired reaction temperature for an appropriate period while azeotroping off the water with a reflux of the azeotroping solvent and at least a partial reflux of the water removed. The reaction may be carried out as a batch, semi-continuous or continuous process. The reaction products are recovered and treated by any conventional method such as stripping of unreacted glycol ether and carboxylic acid and neutralization of the acid catalyst followed by distillation to recover the product ester.

The alkylene glycol monoalkyl ethers which may be employed as reactants in the esterification reaction conform to the general formula $R(OCH_2CHR')_nOH$ and the isomer

$$R(O\underset{|}{\overset{R'}{C}}H{-}CH_2)_nOH$$

as herein above described and which may be prepared for example by the process disclosed in U.S. Patent Nos. 2,816,932 and 4,299,997. Representative alkylene glycol monoalkyl ethers within the above noted formula and suitable for use in this invention include, for example, ethylene glycol and diethylene glycol mono-methyl, -ethyl, -propyl, -isopropyl, -butyl, -tertiary-butyl, -isobutyl, -amyl, -hexyl, -octyl, -decyl, etc., ethers, propylene glycol, dipropylene glycol and tripropylene glycol mono-methyl, -ethyl, -propyl, -isopropyl, -butyl, -amyl, -octyl, -decyl, etc., ethers.

The monocarboxylic acids that may be employed as reactants in the esterification reaction according to the invention include monocarboxylic and halogenated monocarboxylic acids having from 1 to 10 carbon atoms. Representative monocarboxylic acids include for example, formic, acetic, propionic, n-butyric, isobutyric, n-valeric, caproic, pelargonic, trimethylacetic, chloroacetic, bromoacetic, chloroproponic, etc., acids.

The inert azeotroping solvents for use in the process of this invention include those solvents forming a binary minimum-boiling azeotrope with water within the esterification reaction temperature range of from about 80°C. to 165°C. at atmospheric, subatmospheric or superatmospheric pressures. Representative azeotroping solvents include, for example, aromatic hydrocarbons such as benzene, toluene, xylene, chlorobenzene, bromobenzene, alkanes and cycloalkanes such as n-pentane, trimethylpentane, isopentane, n-hexane, n-heptane, nonane, 3,4-dimethylhexane 3-ethylhexane, cyclohexane, methyl cyclohexane, cyclopentane, cyclooctane, halogenated aliphatic compounds such as chloroform, carbon tetrachloride, 1,2-dichloroethane, trichloroethane, etc., as well as diethylketone.

The acid catalysts employed in the present invention are known and include, for example, the mineral acids such as concentrated sulfuric acid, hydrochloric acid, nitric acid, etc., Lewis acids such as boron trifluoride, antimony pentafluoride, etc., organic sulfonic and halogenated sulfonic acids such as methane, ethane, and butane sulfonic acids, trifluoromethane sulfonic acid, trichloromethane sulfonic acid, o- or p-toluene sulfonic acid, benzene sulfonic acid, etc., as well as strongly acidic sulfonated aromatic ion exchange resins and perfluoroalkane sulfonic acid resins. The acid catalysts are generally employed in concentrations of from about 0.01 to 5 weight per cent, preferably 0.1 to 2.0 weight per cent, based on the total reaction mixture, which concentrations within this range vary with the particular acid employed.

The reaction pot temperature of the present invention will range between about 80°C. and 165°C. It is preferred however to operate at temperatures of from 115°C. to 145°C. to obtain a convenient rate of esterification and maintain the water azeotrope. Pot reaction temperatures will of course be based on the azeotroping solvent and pressure, if any, employed in the reaction. The overhead temperatures of the distillation column are, as hereinabove described, maintained between a range of about 65°C. and 95°C. with a reflux of water formed by the reaction. Between about 10 and 95 and preferably between 25 and 75 weight per cent of the water removed is refluxed to the upper portion and trays of the distillation column in order to reduce and maintain the overhead temperature in the distillation column at between about 65°C. and 95°C. until at least near completion, e.g., approximately 80 percent reaction completion and thus depletion of water in the system, at which time the overhead temperature of the distillation column will gradually increase to between about 100°C. to 125°C. which is equivalent to operating in the absence of a water reflux. The process is preferably carried out at atmospheric pressure. However, subatmospheric pressure of about 50 mm Hg up to atmospheric as well as superatmospheric pressures of up to 3.4 bar (5.0 psig) may be used if desirable.

In order to describe the invention in greater detail reference is made to the drawing wherein an alkylene glycol monoalkyl ether, a carboxylic acid, acid catalyst and azeotroping solvent mixture is fed via line 1 to the reactor pot 2 which is then heated either by heating coils 3 within the reactor or by recirculation of the reaction mixture through line 6 via pump 7 to reboiler 5 and through line 4 back into the reactor to maintain the proper reaction temperature. Azeotroping solvent, water and small amounts of unreacted

glycol ether and mono-carboxylic acid as well as some ester product pass into distillation column 8 which is maintained preferably at atmospheric pressure. A predominantly water solvent azeotrope is taken off the top of the distillation column 8 via line 9 to condenser 10. The condensed water-solvent mixture is then fed to phase separator 12 via line 11. The azeotroping solvent-water mixture is separated in phase separator 12. The solvent phase is then removed and refluxed to the distillation column 8 via pump 17 and line 18 while the water phase is taken off and controlled by pump 13 and partially removed via valve 16 through line 14 for discharge and partially refluxed back to the distillation column via valve 15 and line 19.

After completion of the esterification reaction, any unreacted glycol ether and monocarboxylic acid is essentially stripped from the remaining ester product in reactor pot 2 which product is then generally treated with, for example, excess sodium carbonate under agitation to neutralize acid catalyst such as para-toluene sulfonic acid and any free monocarboxylic acid remaining. The organic product phase may, for example, be distilled under vacuum and recovered.

Although the method of the present invention will be directed principally to the preparation of the acetic acid ester of propylene glycol monomethyl ether and dipropylene glycol monomethyl ether it is not intended that the method be limited to such ester preparation and those skilled in the art will recognize that the present method is broadly applicable to the preparation of other esters such as propylene glycol monobutyl acetate, dipropylene glycol monooctyl butyrate, ethylene glycol monoethyl formate, etc., using the appropriate glycol ether and monocarboxylic acid.

The following examples are provided to illustrate the invention in accordance with the principles of this invention but are not to be construed as limiting the invention in anyway except as indicated by the appended claims.

Example 1

A liquid mixture of 400 g. (4.44 mols) of propylene glycol monomethyl ether, 255 g. (4.25 moles) glacial acetic acid, 60 g. toluene and 2.00 g. concentrated sulfuric acid (95.7%) was charged to a 1000 ml 3 neck round bottom reaction flask with a heating mantle and equipped with a thermometer, magnetic stirrer and a 1" diameter 25 tray Oldershaw distillation column with a distilling head and thermometer. The reaction was run for a period of 2 hours at a pot temperature, which was gradually increased, from between 115°C. to 135°C. at the end of the run. Water was removed by water-toluene azeotrope. 30 per cent by weight of water removed during the reaction was refluxed to the top of the distillation column along with total reflux of the toluene to maintain an overhead distillation column temperature of 82°—88°C. which was maintained to within 10 minutes of reaction completion when the temperature increased to 120°C.—125°C. Analysis of the product remaining in the reactor by gas chromatography and titration showed a 90 per cent conversion of the propylene glycol monomethyl ether and a 93 per cent conversion based on the acetic acid. Selectivity to the propylene glycol monomethyl ether acetate was 99+ per cent. Only 2 weight per cent of the propylene glycol monomethyl ether and 0.3 weight per cent of the acetic acid charged to the reactor was lost in the aqueous phase taken overhead.

Example 2 (Comparative)

A mixture of 40 g. of propylene glycol monomethyl ether, 25.5 g. of glacial acetic acid, 20 g. toluene and 0.40 g. concentrated sulfuric acid was charged to similar reaction equipment as used in Example 1 but with no provision for the reflux of water to the distillation unit. The reaction was run for 2 hours at a pot temperature of between 118°C. and 122°C, with water removed overhead with Dean Stark apparatus and the toluene refluxed. The overhead temperature of the distillation column was between 103° and 104°C. during the reaction. Analysis of the product ester showed only 84 per cent conversion of the propylene glycol monomethyl ether and acetic acid. Selectivity based on the propylene glycol monomethyl ether was 95 weight per cent. 8 weight per cent propylene glycol monomethyl ether and 10 weight per cent acetic acid including some product ester was lost in the aqueous phase removed.

Example 3

The procedure of Example 1 was repeated employing 670 g. of dipropylene glycol monomethyl ether, 255 g. glacial acetic acid, 40 g. toluene and 4.6 g. para-toluene sulfonic acid monohydrate as catalyst. The reaction was run for two hours at a pot temperature of between 115°C. and 135°C. with 35 per cent of the total water removed during the reaction being refluxed to the top of the distillation column along with a total reflux of the toluene. The overhead temperature of the distillation column was reduced and maintained at 82°—88°C. to within 10 minutes of completion of the reaction when the temperature of the overhead increased to ∾105°C. Analysis of the product by gas chromatography and titration showed 99 per cent selectivity with an 89 per cent conversion of reactants. Loss of glycol ether and acid reactants to the aqueous phase was only 2.7 weight per cent and 0.5 weight per cent respectively.

Example 4

The procedure of Example 1 was repeated using a reactor charge of 400 g. propylene glycol monomethyl ether, 255 g. glacial acetic acid, 40 g toluene and 3.30 g. p-toluene sulfonic acid monohydrate as catalyst. The reaction was run for 2 hours at a pot temperature, which was gradually increased, from 115°C. to 135°C. toward the end of the run. 30 per cent of the total water removed

during the reaction was refluxed to the overhead portion of the distillation column reducing and maintaining the temperature overhead at about 84°C. to within 10 minutes of completion of the reaction when the temperature increased to ∾103°C. Analysis of product showed a 90 per cent conversion of reactants with 99+ per cent selectivity. Loss of ether and acid reactants to the aqueous phase was only 2 and 0.4 weight per cent respectively.

Example 5 (Comparative)

Example 4 was repeated using the same reactants, as well as the same amount of reactants. The reaction was run for 3 hours at a pot temperature of 116°C—135°C. Water removed during distillation was discharged from the system via Dean Stark apparatus with a reflux of toluene. Without the water reflux the overhead temperature in the distillation column remained at ∾104°C. Analysis of the product by gas chromatograph showed 82 per cent conversion of reactants with 99 per cent selectivity. Loss of the glycol ether and acid reactants was 3.9 and 5.0 weight per cent respectively.

Example 6

The procedure of Example 1 was repeated using a reactor charge of 4000 g of propylene glycol monomethyl ether, a 2400 g. of glacial acetic acid, 1800 g of toluene and 250 g. of sulfonated polyaromatic ion exchange resin (sold, for example, commercially as "Amberlyst 15" by Rohm and Haas Co.) which resin has a bulk density of approximately 595 g/l., a hydrogen ion concentration of approximately 4.9 milliequivalents/g. dry, a surface area of from about 40 to 50 $m^2$/g. and an average pore diameter of from about 200 to 600 Angstrom units. The reaction was run for 4 hours at a reaction pot temperature of 110°C. Approximately 25 per cent of the water removed during azeotropic distillation was refluxed into the overhead portion of the distillation column reducing and maintaining the overhead temperature of the column to 83°—84°C. until toward the end of the reaction and depletion of water in the system when the temperature in the column overhead rose to ∾103°C. Analysis of the product showed 83 per cent conversion with 84 per cent selectivity. Loss of glycol ether and acid reactants were 4.0 and 0.4 respectively.

Example 7 (Comparative)

Example 6 was repeated using the same reactants as well as the same amounts of reactants, except that total water removed during distillation was discharged from the system via Dean Stark apparatus with a total reflux of toluene. Without the water reflux overhead, temperature in the distillation column remained at ∾104°C. during the reaction. Analysis of the product showed an 82 per cent conversion of reactants with 77 per cent selectivity. Loss of glycol ether and acid reactants were 5.0 and 5.0 respectively.

**Claims**

1. A method for the preparation of a monocarboxylic acid ester of an alkylene glycol monoalkyl ether which comprises the steps of:

reacting at a reaction pot temperature of from about 80°C. to 165°C., a monocarboxylic or halogenated monocarboxylic acid having from 1 to 10 carbon atoms with an alkylene glycol monoalkyl ether having the formulae

$$R(OCH_2CHR')_nOH \quad \text{and} \quad R(O\overset{R'}{\overset{|}{C}}HCH_2)_nOH$$

wherein R is a straight or branched chain alkyl group containing from 1 to 10 carbon atoms, R' is hydrogen or methyl group and n is an integer of from 1 to 3, in the presence of an acid catalyst and a water-azeotroping solvent which forms a binary minimum-boiling azeotrope with water within the reaction temperature range;

removing water via a distillation column during said reaction by azeotropic distillation with said water-azeotroping solvent;

separating the water-solvent azeotrope forming a solvent phase and a water phase;

refluxing the solvent phase and a portion of the water phase to the overhead section of the distillation column to reduce and maintain an overhead temperature in said column at between about 65°C. and 95°C.; and

recovering the desired monocarboxylic acid ester.

2. A method according to Claim 1 wherein the monocarboxylic acid is acetic acid.

3. A method according to Claim 1 or Claim 2 characterised in that the alkylene glycol monoalkyl ether is propylene glycol monomethyl ether or dipropylene glycol monomethyl ether.

4. A method according to any one of Claims 1 to 3 characterised in that the acid catalyst is selected from sulfuric acid, p-toluene sulfonic acid and sulfonated polyaromatic ion exchange resins and is preferably p-toluene sulfonic acid.

5. A method as claimed in any one of Claims 1 to 4 characterised in that the reaction temperature is between 115°C and 145°C.

6. A method as claimed in any one of Claims 1 to 5 characterised in that between about 10 and 95 weight percent, and preferably between about 25 and 75 weight percent, of the water phase is refluxed to the overhead section of the distillation column.

7. A method as claimed in any one of Claims 1 to 6 characterised in that the water-azeotroping solvent is toluene.

8. A method as claimed in any one of Claims 1 to 7 characterised in that between about 1 to 25 weight percent, and preferably between about 3 and 10 weight percent, water-azeotroping solvent is employed in the reaction based on the total reaction mixture.

9. A method as claimed in any one of Claims 1 to 8 characterised in that the acid catalyst is

employed in a concentration of from about 0.01 to 5 weight percent, and preferably about 0.1 to 2.0 weight percent, based on the total reaction mixture.

10. A method as claimed in any one of Claims 1 to 9 characterised in that the reaction is carried out uner a superatmospheric pressure of up to 3.4 bar (50 psig).

11. A method for the preparation of propylene glycol monomethyl ether acetate which comprises the steps of

reacting at a reaction pot temperature of from about 115°C to 145°C acetic acid with propylene glycol monomethyl ether, in the presence of p-toluene sulfonic acid catalyst and toluene water-azeotroping solvent;

removing water via a distillation column during said reaction by azeotropic distillation with said toluene;

separating the water-toluene azeotrope forming a water phase and a toluene phase;

refluxing the toluene phase and between about 25 and 75 weight percent of the water phase to the overhead section of the distillation column to reduce and maintain an overhead temperature in said column at between about 82°C and 88°C and

recovering the desired propylene glycol monomethyl ether acetate.

**Patentansprüche**

1. Verfahren zur Herstellung eines Monocarbonsäureesters aus einem Alkylenglykolmonoalkylether, dadurch gekennzeichnet, daß bei einer Temperatur von 80°C bis 165°C im Reaktionskolben eine 1 bis 10 Kohlenstoffatome aufweisende Monocarbonsäure oder eine halogenierte Monocarbonsäure umgesetzt wird, mit einem Alkylenglykolmonoalkylether der Formel

$$R(OCH_2CHR')_nOH \quad \text{und} \quad R(O\underset{\underset{R'}{|}}{C}HCH_2)_nOH$$

worin R eine geradkettige oder verzweigtkettige Alkylgruppe mit 1 bis 10 Kohlenstoffatomen R' ein Wasserstoffatom oder eine Methylgruppe bedeutet, und n eine ganze Zahl von 1 bis 3 ist, in Gegenwart eines sauren Katalysators und eines mit Wasser ein Azeotrop bildenden Lösungsmittels, das ein binäres, tiefsiedendes Azeotrop mit Wasser innerhalb des Reaktionstemperaturbereiches bildet, daß das Wasser während der Reaktion über eine Destillationskolonne mittels azeotroper Destillation mit dem mit Wasser ein Azeotrop bildenden Lösungsmittel entfernt wird, daß das Wasser-Lösungsmittel azeotrop, das eine Lösungsmittelphase und eine Wasserphase bildet, abgetrennt wird, daß die Lösungsmittelphase und ein Teil der Wasserphase unter Rückfluß zu dem Kopfteil der Destillationskolonne erhitzt wird um die Temperatur zu vermindern und zwischen ungefähr 65°C und 95°C aufrechtzuerhalten; und daß der gewünschte Monocarbonsäureester gewonnen wird.

2. Verfahren nach Anspruch 1, dadurch gekennzeichnet, daß die Monocarbonsäure Essigsäure ist.

3. Verfahren nach Anspruch 1 oder 2, dadurch gekennzeichnet, daß der Alkylenglykolmonoalkylether Propylenglykolmonomethylether oder Dipropylenglykolmonomethylether ist.

4. Verfahren nach einem der Ansprüche 1 bis 3, dadurch gekennzeichnet, daß der saure Katalysator ausgewählt ist aus Schwefelsäure, p-Toluolsulfonsäure, einem sulfonierten polyaromatischen Ionenaustauscherharz und daß er vorzugsweise p-Toluolsulfonsäure ist.

5. Verfahren nach einem der Ansprüche 1 bis 4, dadurch gekennzeichnet, daß die Reaktionstemperatur zwischen 115°C und 145°C liegt.

6. Verfahren nach einem der Ansprüche 1 bis 5, dadurch gekennzeichnet, daß zwischen 10 und 95 Gew.-%, vorzugsweise zwischen etwa 25 bis 75 Gew.-%, der Wasserphase unter Rückfluß zu dem Kopfteil der Destillationskolonne erhitzt wird.

7. Verfahren nach einem der Ansprüche 1 bis 6, dadurch gekennzeichnet, daß das mit Wasser ein Azeotrop bildende Lösungsmittel Toluol ist.

8. Verfahren nach einem der Ansprüche 1 bis 7, dadurch gekennzeichnet, daß zwischen ungefähr 1 bis 25 Gew.-% vorzugsweise zwischen ungefährt 3 und 10 Gew.-%, bezogen auf die Gesamtreaktionsmischung, das mit Wasser ein Azeotrop bildende Lösungsmittel in der Reaktion eingesetzt wird.

9. Verfahren nach einem der Ansprüche 1 bis 8, dadurch gekennzeichnet, daß der saure Katalysator in einer Konzentration von ungefähr 0,01 bis 5 Gew.-% vorzugsweise ungefähr 0,1 bis 2,0 Gew.-%, bezogen auf die Gesamtreaktionsmischung, verwendet wird.

10. Verfahren nach einem der Ansprüche 1 bis 9, dadurch gekennzeichnet, daß die Reaktion in einer Überdruckatmosphäre von bis zu 3,4 bar (50 psig) durchgeführt wird.

11. Verfahren zur Herstellung von Propylenglykolmonomethyletheracetat, dadurch gekennzeichnet, daß bei einer Behältertemperatur von ungefähr 115°C bis 145°C Essigsäure mit Propylenglykolmonoethylether in Gegenwart eines p-Toluolsulfonsäurekatalysators und einem mit Wasser ein Azeotrop bildendes Lösungsmittel umgesetzt wird, daß das Wasser während der Reaktion mittels azeotroper Destillation mit Toluol über eine Destillationskolonne entfernt wird, daß das Wasser/Toluol-Azeotrop, das eine Wasserphase und eine Toluolphase bildet, abgetrennt wird, daß die Toluolphase und zwischen ungefähr 25 und 75 Gew.-% der Wasserphase zu dem Kopfteil der Destillationskolonne unter Rückfluß erhitzt wird, um port die Temperatur in der Kolonne zu vermindern und zwischen ungefähr 82°C und 88°C aufrecht zu erhalten, und daß das gewünschte Propylenglykolmonomethyletheracetate gewonnen wird.

**Revendications**

1. Procédé pour la préparation d'un ester

d'acide monocarboxylique d'un éther monoalcoylique d'alcoylène glycol, caractérisé en ce qu'il comprend les étapes de:

réaction à une température de pot de réaction d'environ 80 à 165°C, d'un acide monocarboxylique ou monocarboxylique halogéné ayant de 1 à 10 atomes de carbone avec un éther monoalcoylique d'alcoylène glycol représenté par les formules

$$R(OCH_2CHR')_nOH \quad \text{et} \quad R(O\overset{R'}{\overset{|}{C}}HCH_2)_nOH$$

dans lesquelles R est un groupe alcoyle à chaîne droite ou ramifiée contenant de 1 à 10 atomes de carbone, R' est un atome d'hydrogène ou un groupe méthyle et n est entier de 1 à 3, en présence d'un catalyseur acide et d'un solvant formant un azéotrope avec l'eau, qui forme un azéotrope binaire à point d'ébullition minimum avec l'eau dans la gamme de température réactionnelle;

élimination de l'eau via une colonne de distillation pendant cette réaction, par distillation azéotropique avec ce solvant formant un azéotrope avec l'eau;

séparation de l'azéotrope eau-solvant et formation d'une phase solvant et d'une phase aqueuse;

renvoi de la phase solvant et d'une portion de la phase aqueuse à la section de tête de la colonne de distillation pour réduire et maintenir une température de tête dans cette colonne entre environ 65 et 95°C; et

récupération de l'ester d'acide monocarboxylique désiré.

2. Procédé suivant la revendication 1, caractérisé en ce que l'acide monocarboxylique est l'acide acétique.

3. Procédé suivant la revendication 1 ou 2, caractérisé en ce que l'éther monoalcoylique d'alcoylène glycol est l'éther monométhylique de propylène glycol ou l'éther monométhylique de dipropylène glycol.

4. Procédé suivant l'une quelconque des revendications 1 à 3, caractérisé en ce que le catalyseur acide est choisi parmi l'acide sulfurique, l'acide p-toluène sulfonique et des résines d'échange d'ion polyaromatiques sulfonées et qu'il est de préférence l'acide p-toluène sulfonique.

5. Procédé suivant l'une quelconque des revendications 1 à 4, caractérisé en ce que la température de réaction est comprise entre 115 et 145°C.

6. Procédé suivant l'une quelconque des revendications 1 à 5, caractérisé en ce qu'entre environ 10 et 95% en poids et de préférence qu'entre environ 25 et 75% en poids de la phase aqueuse sont renvoyés dans la section de tête de la colonne de distillation.

7. Procédé suivant l'une quelconque des revendications 1 à 6, caractérisé en ce que le solvant formant un azéotrope avec l'eau est le toluène.

8. Procédé suivant l'une quelconque des revendications 1 à 7, caractérisé en ce qu'entre environ 1 et 25% en poids et de préférence qu'entre environ 3 et 10% en poids de solvant formant un azéotrope avec l'eau, sont employés dans la réaction par rapport au mélange réactionnel total.

9. Procédé suivant l'une quelconque des revendications 1 à 8, caractérisé en ce que le catalyseur acide est employé en une concentration d'environ 0,01 à 5% en poids et de préférence d'environ 0,1 à 2,0% en poids par rapport au mélange réactionnel total.

10. Procédé suivant l'une quelconque des revendications 1 à 9, caractérisé en ce que la réaction est effectuée sous une pression superatmosphérique allant jusqu'à 3,4 bars (50 psig).

11. Procédé pour la préparation d'acétate d'éther monométhylique de propylène glycol, caractérisé en ce qu'il comprend les étapes de:

réaction à une température de pot de réaction d'environ 115 à 145°C, d'acide acétique et d'éther monométhylique de propylène glycol, en présence d'acide p-toluène sulfonique comme catalyseur et de toluène comme solvant formant un azéotrope avec l'eau;

élimination de l'eau via une colonne de distillation pendant cette réaction par distillation azéotropique avec ce toluène;

séparation de l'azéotrope eau-toluène et formation d'une phase toluénique et d'une phase aqueuse;

renvoi de la phase toluénique et d'environ 25 à 75% en poids de la phase aqueuse à la section de tête de la colonne de distillation pour réduire et maintenir une température de tête dans cette colonne entre environ 82 et 88°C; et

récupération de l'acétat d'éther monométhylique de propylènce glycol désiré.

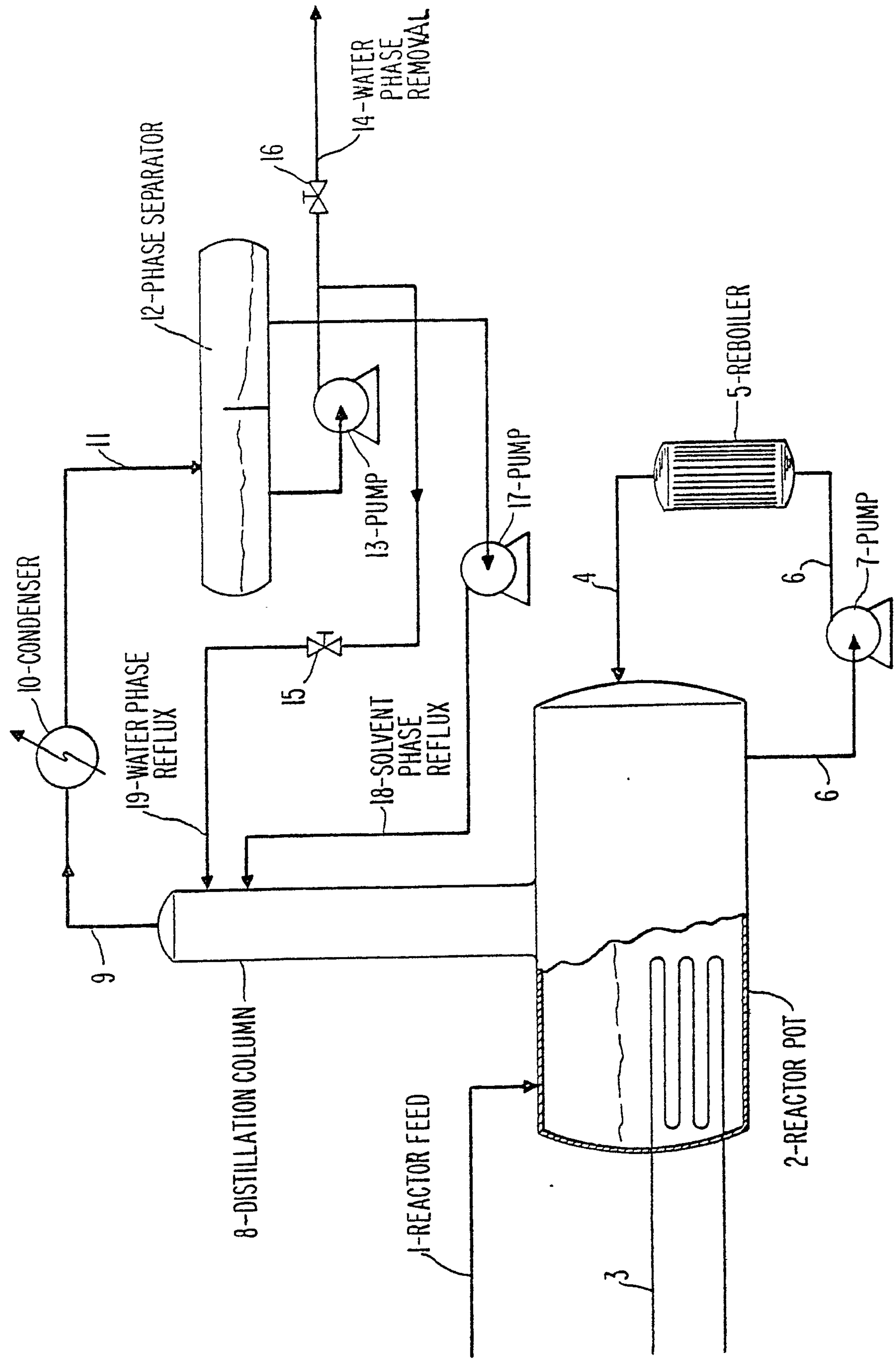

14-WATER PHASE REMOVAL
16
12-PHASE SEPARATOR
11
10-CONDENSER
13-PUMP
17-PUMP
5-REBOILER
19-WATER PHASE REFLUX
15
18-SOLVENT PHASE REFLUX
4
6
7-PUMP
6
9
8-DISTILLATION COLUMN
1-REACTOR FEED
2-REACTOR POT
3